# EUROPEAN PATENT APPLICATION

(11) **EP 4 297 042 A1**
(43) Date of publication of application: **27.12.2023**
(21) Application number: 22187707.9
(22) Date of filing: 29.07.2022
(51) Int. Cl.: G16H 30/40, G16H 50/20, G16H 50/30

(54) **HEAD MOUNTED MEDICAL DEVICE AND OPERATING METHOD**

(30) Priority: 24.06.2022 CN 202210722753
(71) Applicant: Vivest Medical Technology Co., Ltd., Suzhou, Jiangsu 215123 (CN)
(72) Inventor: Zheng, Jie, Suzhou, 215123 (CN); Xu, Haishan, Suzhou, 215123 (CN)
(74) Representative: Winter, Brandl - Partnerschaft mbB

(57) **Abstract**

A head mounted medical device and an operating method are provided. The medical device includes an information acquisition module (M10) for acquiring physiological sign information of a patient and/or operation information; a remote communication module (M20) for communicating with an external wireless communication module, communicating includes transmitting a data collected by the information acquisition module (M10) to the external wireless communication module and receiving a data sent by the external transmission module; an information output module (M30) for displaying or sending the data sent by the external wireless communication module to the person wearing the medical device. The medical device can be used in emergency situations and can assist the rescuer wearing it to automatically obtain the patient's physiological status, automatic diagnosis from the device or remote diagnosis, remote guidance and tips etc., which greatly standardize the operations of first aid and thus improves the success rate of first aid.

## Description

### CROSS-REFERENCE TO RELATED APPLICATIONS

This application is based upon and claims priority to Chinese Patent Application No. 202210722753.8, filed on June 24, 2022, the entire contents of which are incorporated herein by reference.

### TECHNICAL FIELD

The present disclosure relates generally to the field of medical devices and more particularly to a head mounted medical device and an operating method.

### BACKGROUND

According to the "China Cardiovascular Health and Disease Report 2019", the number of sudden cardiac death (SCD) cases in China is as high as 544,000 per year. When an SCD patient suffers cardiac arrest, timely implementation of high-quality and effective cardiopulmonary resuscitation (CPR) is the key step to rescue the SCD patient. Chest compression is one of the core processes in CPR process. The quality of chest compression is directly related to the success rate of rescue and survival rate of the patients. Some authoritative rescue guidelines have recommended specific treatment parameters for CPR process and chest compression. For example, with years of accumulated research and clinical data statistics, the American Heart Association(AHA) CPR guidelines recommend that during chest compressions, the compression depth should be no less than 5cm and no more than 6cm; the compression rate should be between 100-120 (times/minute); interruptions in compressions should be minimized; compressions should be released completely, that is, the chest should be fully released to zero depth after each compression before starting the next compression. Therefore, in clinical practice, monitoring and feedback of these parameters is the key to improve the quality of CPR process and the success rate of rescue.

On the other hand, the occurrence of SCD is accidental; the scene environment is complex; the skills and capabilities of the rescuers are varied; some social factors, such as imperfect laws, cause bystanders to be less willing to rescue, especially for some non-professional bystanders who lack confidence in their own rescue ability. These often cause SCD patients to miss the best rescue opportunity.

Therefore, if the scene can be recorded and stored, and the rescuer can be remotely assisted, such as providing rescue guidance, intelligent collection and diagnosis of patient status, information transmission and sharing, CPR monitoring and feedback, which will greatly remove the hindrance to providing rescue when SCD occurs.

### SUMMARY

In order to overcome the current technical hurdles, the present disclosure provides a head mounted medical device and an operating method. The head mounted medical device can be used in emergency situations. For example, when a patient suddenly has an acute heart attack, a rescuer may wear the medical devices, and the medical device can assist the rescuer wearing it to automatically obtain the patient's physiological status, automatic diagnosis from the device or remote diagnosis, remote guidance and tips etc., which greatly standardize the operations of first aid and thus improves the success rate of first aid.

The present disclosure provides a head mounted medical device including: an information acquisition module for acquiring physiological sign information of a patient and/or operation information of a person wearing the medical device; a remote communication module for communicating with an external wireless communication module, wherein, the communicating includes transmitting a data collected by the information acquisition module to the external wireless communication module and receiving a data sent by the external transmission module; an information output module for displaying or sending the data sent by the external wireless communication module to the person wearing the medical device.

In some embodiments of the present disclosure, the physiological sign information includes one or more of data of thoracic rise and fall, pupil status and skin status; the operation information includes one or more of data of depth of compressions, rate of compressions, incomplete recoil of compressions, duration of compression interruption and duration of compressions.

In some embodiments of the present disclosure, the information acquisition module includes a distance measurement module.

In some embodiments of the present disclosure, the information acquisition module includes an image capture module and/or an audio capture module.

In some embodiments of the present disclosure, the medical device further includes: a diagnosis module, configured to receive the data collected by the information acquisition module and make a diagnosis and/or give a treatment plan according to the data.

In some embodiments of the present disclosure, the diagnosis module includes a first algorithm model and/or a trained second algorithm model; the information acquisition module further includes an image capture module; the first algorithm model is configured to determine whether a patient has cyanosis according to at least one skin image of the patient collected by the image capture module; the second algorithm model is configured to determine whether a patient is in a status of pupil dilation according to at least one eyeball image of the patient collected by the image capture module.

In some embodiments of the present disclosure, the information acquisition module includes a positioning module, which is configured to capture geographic location information of the person wearing the medical device.

In some embodiments of the present disclosure, the information output module includes an image output module and/or an audio output module.

In some embodiments of the present disclosure, the information output module further includes a warning module, which is configured to send warning information by touching a body of the person wearing the medical device.

In some embodiments of the present disclosure, the medical device further includes a lighting module for providing a light source.

In some embodiments of the present disclosure, the medical device further includes a visual indication module for indicating the location of the data collected by the information acquisition module.

In some embodiments of the present disclosure, the remote communication module is also configured to transmit the data to an external storage module according to a pre-set period.

In some embodiments of the present disclosure, the medical device is a device in the form of glasses; the medical device in the form of glasses includes lenses and temples; the information acquisition module includes a distance measurement module, and the distance measurement module includes at least one electromagnetic wave distance measurement device, and the at least one electromagnetic wave distance measurement device is arranged around the lenses; and/or the information acquisition module includes an image capture module, and the image capture module includes two cameras, and the cameras are respectively arranged around the lenses; and/or the information acquisition module includes an audio capture module, and the audio capture module includes at least one sound pickup, and the at least one sound pickup is arranged on the temples or around the lenses.

In some embodiments of the present disclosure, the information output module includes an image output module, the image output module includes a display, the display is moved to the position of a lens when the medical device outputs an image; and/or the information output module includes an audio output module, and the audio output module includes at least one speaker, and the speaker is arranged on the temples; and/or the information output module includes a warning module, and the warning module includes at least one vibrator, and the at least one vibrator is arranged on the temples.

The present disclosure also provides a method for operating a head mounted medical device, including steps of: the medical device acquiring the physiological sign information of a patient and/or the operation information of a person wearing the medical device; the medical device sending the physiological sign information and/or the medical operation information to an external wireless communication module; the medical device receiving the data sent by the external wireless communication module, and displaying or sending the data to the person wearing the medical device.

In some embodiments of the present disclosure, the information acquisition module includes a distance measurement module, and the distance measurement module includes at least one electromagnetic wave distance measurement device; the physiological sign information includes a data of thoracic rise and fall, and the step of acquiring the physiological sign information of a patient by the medical device includes: acquiring positions H of a thorax of the patient at a plurality of moments in a period by using the at least one electromagnetic distance measurement device; obtaining a data of thoracic rise and fall during the period according to positions H of a thorax of the patient.

In some embodiments of the present disclosure, the distance measurement module includes a first electromagnetic wave distance measurement device and a second electromagnetic wave distance measurement device; a position HI of a thorax of the patient and a position H2 of a thorax of the patient at a moment are respectively acquired through the first electromagnetic wave distance measurement device and the second electromagnetic wave distance measurement device; a position H of a thorax of the patient at the moment is an average value of the position HI and the position H2.

In some embodiments of the present disclosure, after obtaining a data of thoracic rise and fall during the period, the method further includes the following steps: acquiring two positions of the thorax of the patient at two adjacent moments from the data of thoracic rise and fall; determining whether a difference between the two positions is greater than a first threshold; if it is greater than the first threshold, the patient is considered to be in a status of spontaneous breathing; if it is not greater than the first threshold, the patient is considered to be in a status of cardiac arrest.

In some embodiments of the present disclosure, when a person wearing the medical device performs CPR on a patient, the method further includes: acquiring a data of thoracic rise and fall during CPR; obtaining one or more of data of depth of compressions, rate of compressions, incomplete recoil of compressions, duration of compression interruption and total duration of compressions according to the data of thoracic rise and fall during CPR.

In some embodiments of the present disclosure, the information acquisition module includes an image capture module, and the image capture module includes at least one camera; the physiological sign information include s a data of thoracic rise and fall, and the step of acquiring the physiological sign information of a patient by the medical device includes: acquiring a plurality of thoracic images of a patient in a period; obtaining a data of thoracic rise and fall during the period according to the plurality of thoracic images.

In some embodiments of the present disclosure, the information acquisition module includes an image capture module, and the image capture module includes at least one camera; the physiological sign information includes a data of pupil status, and the step of acquiring the physiological sign information of a patient by the medical device includes: acquiring at least one image of eyeball of a patient in a period; obtaining a pupil-to-eyeball ratio of the patient according to the at least one image of eyeball.

In some embodiments of the present disclosure, the medical device further includes a visual indication module, and the visual indication module emits a beam spot, the method further includes: acquiring at least one image of the beam spot and at least one image of eyeball of a patient; obtaining a size of a pupil and a size of a eyeball, and/or pupil-to-eyeball ratio of the patient according to at least one image of the beam spot and the at least one image of eyeball.

In some embodiments of the present disclosure, the information acquisition module includes an image capture module, and the image capture module includes at least one camera; the medical device further includes a diagnosis module, and the diagnosis module stores a skin status database including a mapping relationship between a skin image and a skin status; the physiological sign information includes skin status data; the step of acquiring the physiological sign information by the medical device includes: obtaining at least one skin image of a patient; obtaining skin status data of the patient according to the at least one skin image information through the diagnosis module.

In some embodiments of the present disclosure, the information acquisition module further includes a positioning module, which is configured to capture geographic location information of the person wearing the medical device; the method further includes: sending the geographic location information to the external wireless communication module.

The head mounted medical device in the present disclosure shows the following advantages:
1. Collaboration, data sharing, rescue support between the rescue site and remote medical institutions through the remote communication module provide professional rescue guidance to the rescuers on-site, making the rescuers more confident or willing to rescue, which will advance the rescue time and improve the success rate of SCD rescue.
2. The medical device can synchronize feedback the CPR compression of the rescuer wearing it, and provide CPR compression guidance to the rescuer, so that the compression operation meets the requirements of the guidelines. The above guidance combined with remote diagnosis will give patients the CPR treatment with high quality.
3. The medical device can automatically identify the patient's physiological status or transmit the patient's physiological status in real time. Therefore, it can be determined whether the patient has spontaneous breathing, whether the patient's pupil is enlarged, and whether the patient's skin has cyanosis through automatic diagnosis or remote diagnosis. It can further confirm whether the patient is in the dangerous stage of SCD, so that the rescuer and the remote terminals can confirm the patient's status and then determine the optimal rescue plan as soon as possible.
4. Through the remote communication module and the positioning module, the patient's location information can be quickly identified. When the patient's location is identified, the delivery of physical objects and resources, such as the delivery of automated external defibrillators (AEDs) by drones, or the dispatch of ambulances and personnel. etc., can be carried out in the future, which will greatly reduce waiting time of patients and thereby increase the success rate of rescues.
5. The head mounted structure of the medical equipment liberates the rescuer's hands while providing the patient's physiological status information, so that the rescuer can concentrate on obtaining the information of on-site monitoring and the remote guidance, and then carry out CPR, breathing, defibrillation or other rescue operations, which will greatly reduce the interruption time of CPR.
6. Through the recording and remote storage of on-site information, the medical malpractice disputes caused by social factors, such as imperfect laws, can be avoided, which makes the rescuers willing to carry out rescue and then greatly increases the number of people who are willing to provide rescue.
7. Remote storage of on-site information and analysis/statistics of this information can reconstruct the complete rescue process, which will be beneficial to the development and optimization of rescue operations.

In conclusion, the head mounted medical device in the present disclosure can be used in emergency situations. For example, when a patient suddenly has an acute heart attack, a rescuer may wear the medical devices, and the medical device can assist the rescuer wearing it to automatically obtain the patient's physiological status, automatic diagnosis from the device or remote diagnosis, remote guidance and tips etc., which greatly standardize the operations of first aid and thus improves the success rate of first aid.

### BRIEF DESCRIPTION OF THE DRAWINGS

In order to more clearly illustrate technical solutions in the embodiments of the present disclosure, the drawings used in the description of the embodiments are briefly described below. Obviously, the drawings in the following description are merely some embodiments of the present disclosure. Those skilled in the art can also obtain other drawings based on these drawings without any creative labor.
FIG. 1 is a structural block diagram of a head mounted medical device according to an embodiment of the present disclosure.
FIG. 2 is a schematic diagram of a head mounted medical device according to an embodiment of the present disclosure.
FIG. 3 is a side view of a head mounted medical device according to an embodiment of the present disclosure.
FIG. 4 is a schematic diagram of a head mounted medical device according to another embodiment of the present disclosure.
FIG. 5 is a usage scenario of a head mounted medical device according to another embodiment of the present disclosure.
FIG. 6 is another usage scenario of a head mounted medical device according to another embodiment of the present disclosure.
FIG. 7 is a flowchart of a method for operating a head mounted medical device according to another embodiment of the present disclosure.
FIG. 8 is another usage scenario of a head mounted medical device according to an embodiment of the present disclosure.
FIG. 9 is a graph of thoracic rise and fall data of a patient according to another embodiment of the present disclosure.
FIG.10 and FIG. 11 are respectively usage scenarios of a head mounted medical device according to various embodiments of the present disclosure.

### DETAILED DESCRIPTION

To better illustrate the purpose of the present disclosure, technical proposal and advantages thereof, embodiments of the present disclosure will be described in detail with reference to the drawings. It should be readily understood that both the embodiments and the drawings are explanatory for the present disclosure only, and are not intended as a limitation on the scope of the present disclosure.

In order to improve rescue efficiency and improve rescue success rate, a head mounted medical device and operating method are provided. The medical device includes an information acquisition module for acquiring physiological sign information of a patient and/or operation information of a person wearing the medical device; a remote communication module for communicating with an external wireless communication module, including transmitting a data collected by the information acquisition module to the external wireless communication module and receiving a data sent by the external transmission module; an information output module for displaying or sending the data sent by the external wireless communication module to the person wearing the medical device. The head mounted medical device in the present disclosure can be used in emergency situations. For example, when a patient suddenly has an acute heart attack, a rescuer may wear the medical devices, and the medical device can assist the rescuer wearing it to automatically obtain the patient's physiological status, automatic diagnosis from the device or remote diagnosis, remote guidance and tips etc., which greatly standardize the operations of first aid and thus improves the success rate of first aid.

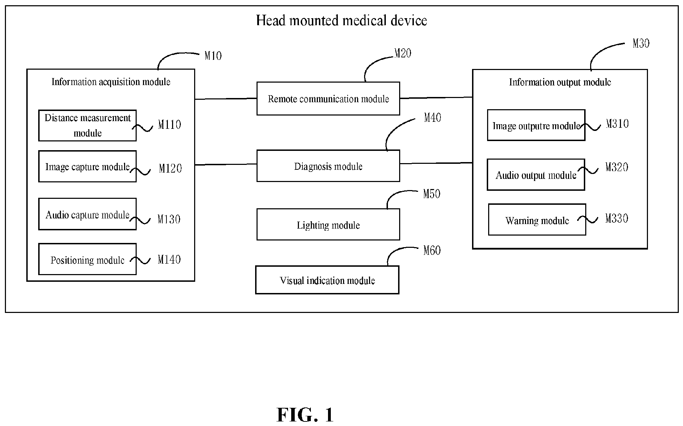

FIG. 1 shows a structural block diagram of a head mounted medical device according to an embodiment of the present disclosure, Specifically, a head mounted medical device includes:
an information acquisition module M10, which is configured to acquire physiological sign information of a patient and/or operation information of a person wearing the medical device. The information acquisition module M10 may include a distance measurement module M110, an image capture module M120 and/or an audio capture module M130. Through the distance measurement module M110, the image capture module M120 and the audio capture module M130 etc., physiological sign information that can be acquired by the information acquisition module M10 may include one or more of data of thoracic rise and fall, pupil status and skin status; and when a person wearing the medical device performs CPR on a patient, operation information of the person that can be acquired may include one or more of data of depth of compressions, rate of compressions, incomplete recoil of compressions, duration of compression interruption and total duration of compressions. Of course, through the image capture module M120 and the audio capture module M130, on-site audio or video can also be acquired.

In some embodiments, the information acquisition module M10 may further include a positioning module M140 for capturing geographic location information of the person wearing the medical device. The location of the rescue site can be shared through the positioning module M140, and the remote end can control the dispatch substances accordingly, such as dispatching AEDs to the scene by drones.

The head mounted medical device further includes a remote communication module M20, which is configured to communicate with an external wireless communication module. Communication includes transmitting a data collected by the information acquisition module to the external wireless communication module and receiving a data sent by the external transmission module. The remote communication module M20 realizes the real-time sharing of the data collected by the medical device to the remote end. The data collected by the medical device is not limited to the above-mentioned data of a patient's physiological sign information and operation information of a person wearing the medical device. The remote end can be a specific medical center or a specific specialist, etc. The remote communication module M20 can also be configured to transmit data to an external storage module according to a pre-set period, and the data of the above-mentioned external storage module can include the physiological sign information of a patient and the operation information, that is, the information of the complete rescue process. The processing and analysis of the above information is beneficial to further perfecting and standardizing the steps of first aid and improving the success rate of first aid. It should be noted that the information transmitted by the remote communication module M20 to the external wireless communication module is not limited to the patient's physiological sign information and the operation information and the like. The records of the first rescue scene, such as images and videos acquired by the image capture module M120 and the audio capture module M130 can be also transmitted.

The head mounted medical device further includes an information output module M30, which is configured to display or send the data sent by the external wireless communication module to the person wearing the medical device. The information output module M30 may include an image output module M310 and an audio output module M320, etc. The information output by the information output module M30 may be the diagnostic opinion of a specific medical center at the remote end or a specific expert at the remote end, or the guidance of emergency operations, etc. The information output may be images, text, and videos output by the image output module M310, or may be sounds output by the audio output module M320, or a combination of the above two. In some embodiments, the information output module M30 may further include a warning module, which is configured to send warning information by touching a body of the person wearing the medical device. In emergency situations, the rescuer is likely to miss some important instructions or information. Therefore, it is necessary to remind the rescuer through vibration and other perceptible signals. The output information of the information output module M30 can be a combination of vision, hearing and touch.

In some other embodiments, the medical device may further include a lighting module M50, the lighting module M50 may be used to light the environment, so that the medical device wearer can operate conveniently. In addition, the medical device may include a visual indication module M60. The visual indication module M60 may include a laser emission device, and the laser beam emitted can be used to indicate the location of the data collected by the information acquisition module.

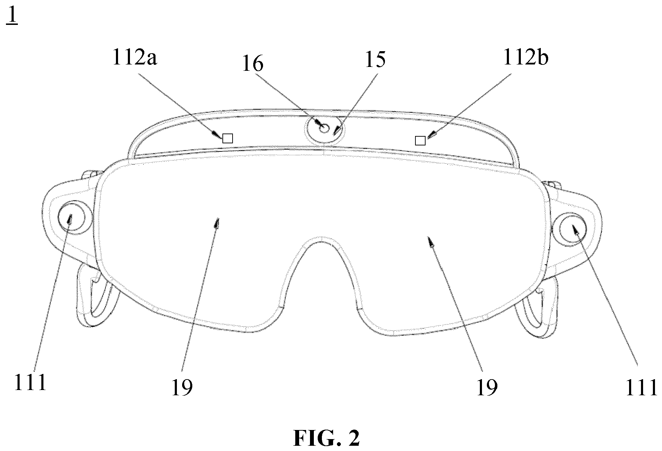

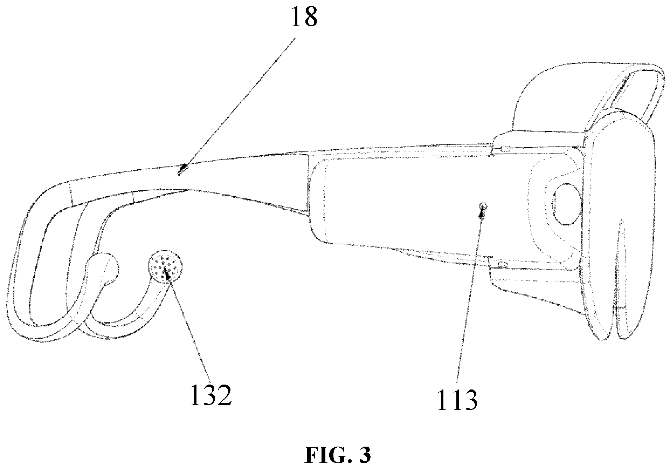

FIG. 2 and FIG. 3 are respectively a schematic diagram and a side view of a head mounted medical device according to an embodiment of the present disclosure. In this embodiment, the medical device 1 is a device in the form of glasses. The medical device 1 in the form of glasses includes lenses and temples. The information acquisition module includes an image capture module and the image capture module includes two cameras 111. The cameras 111 are respectively arranged on two sides of the lenses 19 to capture images or videos in front of the wearer. For example, it can collect on-site images, collect skin images and eyeball images of the patient. The two cameras 111 can be respectively arranged at any positions around the lenses of the medical device, such as arranged at positions above the lenses.

In this embodiment, the information acquisition module 1 includes a distance measurement module, and the distance measurement module includes at least one electromagnetic wave distance measurement device, and the at least one electromagnetic wave distance measurement device is arranged positions above the lenses. For example, 112a and 112b in FIG. 2 may be an electromagnetic wave transmitting unit and an electromagnetic wave receiving unit of an electromagnetic wave distance measurement device, respectively. Preferably, the electromagnetic wave transmitting unit is a radar device in a millimeter-wave, and the electromagnetic wave can be a millimeter-wave with a frequency of 60 GHz.

In some other embodiments, the distance measurement module may include two electromagnetic wave distance measurement devices, one electromagnetic wave distance measurement device of a certain frequency can be set at the position of 112a, and another electromagnetic wave distance measurement device of another frequency can be set at the position of 112b. The accuracy of the distance measured by the medical device of the present disclosure can be corrected by setting up a plurality of electromagnetic wave distance measurement devices. Therefore, the number and position of the distance measurement modules configured on the medical equipment are not limited here, and can be designed according to the shape and actual needs of the medical device.

In addition, in this embodiment, the information acquisition module M10 includes an audio capture module M130, and the audio capture module M130 includes at least one sound pickup and the at least one sound pickup is arranged on the temple. As illustrated in FIG. 3, each sound pickup (can be a microphone) 113 can be arranged on one temple 18 for recording the on-side voice or collecting the wearer's voice, etc. Of course, at least one microphone can be arranged at any positions around the lenses of the medical device.

In above embodiment, the information output module M30 may include an image output module M310, and the image output module M310 includes a display. In this embodiment, one lens 19 may be a transparent display, that is, the medical device wearer can see through the lens for direct observation, at the same time, the lens 19 itself can be used as an image output device, which can display all the information that the medical device needs to display and does not affect the wearer's sight when the information is not displayed. Further, to meet the needs of various wearers of the medical device, the lenses 19 can also be customized lenses with vision correction function.

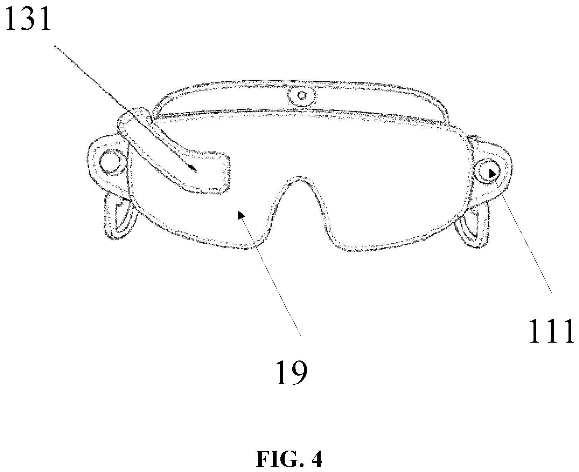

In other embodiments, the display of the image output module can also be movably arranged on the medical device. FIG. 4 is a schematic diagram of a head-mounted medical device according to another embodiment of the present disclosure, wherein a display 131 of the image output module can be rotatably connected to a temple 18. When the medical device outputs an image, the display can be rotated and then moved to the position of the lens 19; when no image output is required, it can be removed to the top of the lens 19 so as not to affect the wearer's sight.

In the embodiment of FIG. 4, there may be a problem that the displayed texts, images, videos and other information are not clear due to the small size of the display 131. In other embodiments, the display 131 of the image output module may be a virtual reality (VR) display device. In this case, the texts, images, videos and other information to be output and be enlarged through the VR display device, which is equivalent to projecting the output texts, images, videos and other information to a certain position in the front of the medical equipment wearer.

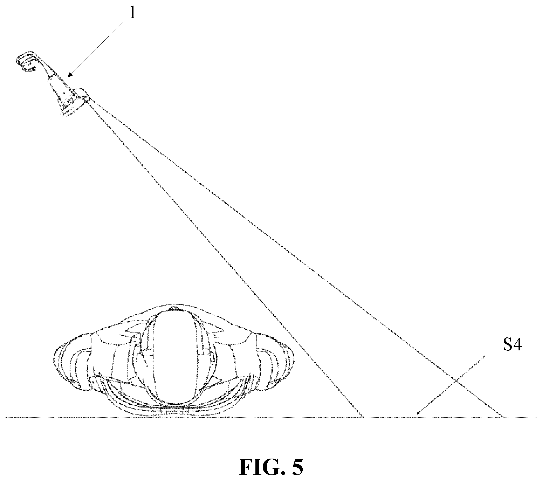

It should be understood that the image output module configured on the medical device of the present disclosure can also be a projection device, and the wearer can directly project the output texts, images, video and other information through the projection device. As shown in FIG. 5, the medical device 1 can project the texts, images, videos and other information to a ground S4 in front of the wearer. The present disclosure does not limit the specific form of the image output module, as long as the output information can be displayed.

In this embodiment, the information output module M30 includes an audio output module M320. The audio output module M320 includes at least one speaker, and the at least one speaker is arranged on the temples of the glasses. In the embodiment of FIG. 3, the ends of the temples 18 may be provided with speakers 132, such as earphones, etc. The ends of the temples 18 may be curved arcs that can fit around the ears of the wearer, so that when the wearer wears the medical equipment, the speaker 132 is near the wearer's ears. The curved ends of the temples 18 can be made of a flexible, deformable material, such as flexible silicone rubber. When the wearer wears the medical device, the shape of the curved ends can be adjusted, so that the medical device of the present disclosure can be applied to various wearers, or the various wearers can wear it more comfortably. The audio output module M320 can also output medical information or warning information to the wearer in the form of voice.

At the same time, the information output module M30 of the medical device may further include a warning module, and the warning module includes at least one vibrator, and the at least one vibrator is arranged on the temples that is in contact with the wearer. When the rescuer needs to be reminded, the vibrator generates vibration, and the vibration can be issued according to a remote command, or it can be an alarm message issued by the medical device itself.

Usually, the head mounted medical device of the present disclosure may be used in various environments, such as an environment with poor lighting. In some embodiments, the head mounted medical device is further provided with a lighting module, and the lighting module can include an LED spotlight 16 as shown in FIG. 2, which is arranged above the lenses 19. The lighting module may also include a plurality of LED lights arranged in a circle. The medical device equipped with the auxiliary lighting modules will be very convenient for emergency operations at night.

A visual indication module of the medical device can include a laser emitting device 15, and the laser emitted can indicate the position of the data collected by the information acquisition module. The laser emitting device 15 can be arranged on the central axis of the two lenses 19. When the laser beam is irradiated on a patient, a mark (laser beam spot) such as a red or green laser beam spot, etc., is displayed on the patient. The marker can show the center position of the light emitted by the lighting module, or can indicate the position of the acquired image.

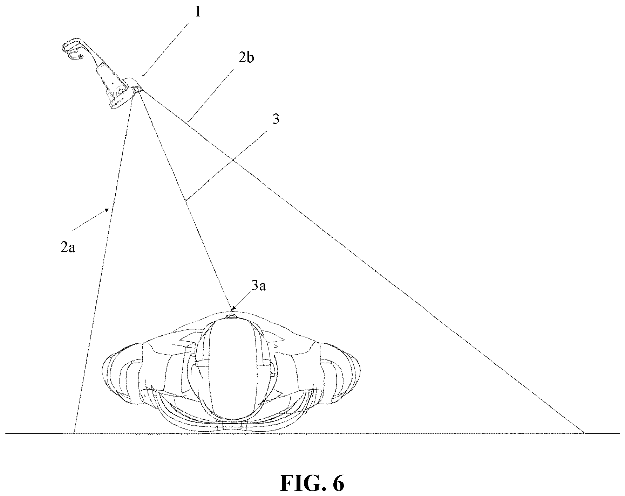

FIG. 6 is another usage scenario of a head mounted medical device according to another embodiment of the present disclosure, wherein, the boundaries 2a and 2b of the light provided by the lighting module, and the emission path 3 of the marker 3a emitted by the visual indication module is indicated when a rescuer wears the head mounted medical device. In the case that chest compression is required, the marker 3a may be a position of the patient where needs to be compressed during CPR first aid. It can be understood that when the rescuer moves, the head mounted medical device will also move, and then the lighting area, the area where the image is captured, and the position monitored by the distance measurement module will also change accordingly. At this point, the marker of the visual indication module can guide the rescuer to direct the head mounted medical device on a specific position and direction.

Further, the medical device of the present disclosure may further include a diagnosis module M40, which is configured to receive the data collected by the information acquisition module and then make a diagnosis and/or give a treatment plan according to the data. For example, in some embodiments, the diagnosis module M40 may include a first algorithm model and/or a trained second algorithm model, the information acquisition module further includes an image capture module. The first algorithm model is configured to determine whether a patient has cyanosis according to at least one skin image of the patient collected by the image capture module; and the second algorithm model is configured to determine whether a patient is in a status of pupil dilation according to at least one eyeball image of the patient collected by the image capture module. The medical device of the present disclosure can intelligently identify the patient's symptoms, such as identifying whether a patient has spontaneous breathing, whether a patient is in a status of cardiac arrest, whether the patient's pupils are dilated, or whether the patient has cyanosis, through a patient's physiological sign information collected by the information acquisition module. At the same time, the first aid operations corresponding to the patient's symptoms can be given. For example, when it is confirmed that the patient is in a critical danger stage of SCD, the medical device will send instructions to the rescuer to perform CPR first aid, and output the specific steps of CPR first aid to the rescuer through the image output module and/or the audio output module. In the absence of remote indications, the diagnostic module of the medical device allows rescuers to still receive guidance and assistance related to the emergency treatment from the medical device.

The medical device disclosed in this disclosure can acquire patient's information, and at the same time can feed back the rescuer's operation information in first aid. In addition, external resources can be dispatched, such as AEDs dispatched by drones etc. Under emergency conditions such as fire suffocation, car accident, etc., the medical equipment disclosed in this disclosure can easily and quickly establish interactive medical support with the remote end professional medical team. At the same time, its records on the first rescue scene can also help to resolve possible medical disputes. Besides being suitable for SCD, the medical device is also suitable for any emergency conditions, such as conscious syncope, acute trauma, drowning, fire suffocation, or car accident. The medical device disclosed in the present disclosure can conveniently and quickly establish interactive medical support with the professional medical team at the remote end. At the same time, its records on the first aid can also help to resolve possible medical disputes.

It should be particularly noted that the positions of the above-mentioned functional modules can be adjusted according to the various forms of the medical devices. The functions of each module of the head mounted medical device can be demonstrated by the following steps in a method of operating the head mounted medical device. The functions of the above mentioned modules are further demonstrated below through the description of a method of operating the head mounted medical device.

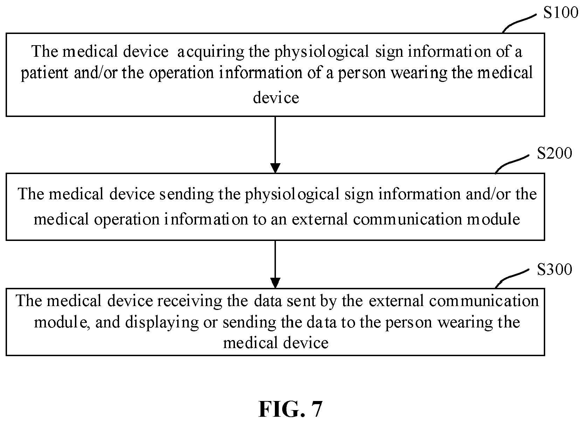

FIG. 7 is a flowchart of a method for operating a head mounted medical device according to another embodiment of the present disclosure. The method is applicable to operate the head mounted medical device, and specifically includes the following steps:
S100: the medical device acquiring the physiological sign information of a patient and/or the operation information of a person wearing the medical device;
S200: the medical device sending the physiological sign information and/or the medical operation information to an external wireless communication module;
S300: the medical device receiving the data sent by the external wireless communication module, and displaying or sending the data to the person wearing the medical device.

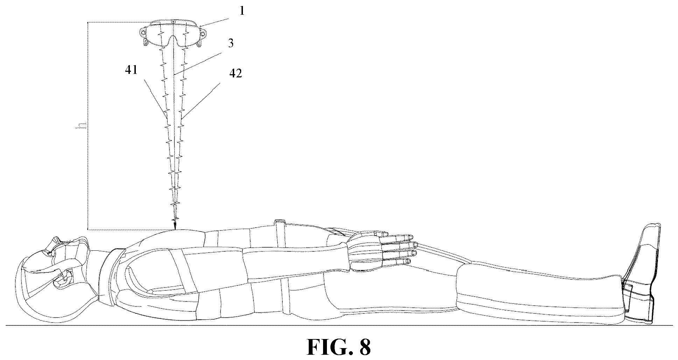

FIG. 8 is another usage scenario of a head mounted medical device according to an embodiment of the present disclosure. In this embodiment, the medical device 1 is in the form of glasses, and the information acquisition module includes a distance measurement module. The distance measurement module includes an electromagnetic wave distance measurement device, and the electromagnetic wave distance measurement device includes an electromagnetic wave transmitting unit and an electromagnetic wave receiving unit. As shown in FIG. 2, the electromagnetic wave transmitting unit 112a and the electromagnetic wave receiving unit 112b can be respectively arranged above the lenses of the medical device, and they are symmetrically arranged with respect to a central axis of the two lenses. In this case, when the electromagnetic wave 41 emitted by the electromagnetic wave transmitting unit 112a contacts a patient's body, the patient's body reflects the electromagnetic wave 42, and the electromagnetic wave receiving unit 112b receives the reflected electromagnetic wave 42. The relative distance between the reflection point on the patient's body and the medical device can be calculated by combining the time difference between the electromagnetic wave transmitting unit 112a transmitting and the electromagnetic wave receiving unit 112b receiving the electromagnetic wave and physical locations of both the electromagnetic wave transmitting unit and the electromagnetic wave receiving unit. When this reflection point of the electromagnetic wave is the sternum center of a patient's thorax, the relative distance between the sternum center of the thorax and the medical device can be obtained by recording the relative distances between the reflection point and the medical device at various consecutive times, that is, positions H of the patient's thorax at a plurality of moments in one period can be obtained. According to the positions H of the patient's thorax at each moment, a data of thoracic rise and fall during the period is obtained. The electromagnetic wave transmitting unit 112a and the electromagnetic wave receiving unit 112b may be arranged at any position around the lenses of the medical device, for example, respectively on two sides of the lenses of the medical device.

Therefore, a data of thoracic rise and fall of a patient can be a waveform diagram of the thoracic position that changes over time. After obtaining a data of thoracic rise and fall during the period, the method may further include the following steps:
acquiring two positions of the thorax of the patient at two adjacent moments from the data of thoracic rise and fall;
determining whether a difference between the two positions is greater than a first threshold;
if it is greater than the first threshold, the patient is considered to be in a status of spontaneous breathing;
if it is not greater than the first threshold, the patient is considered to be in a status of cardiac arrest. The first threshold here may be empirical data obtained clinically.

It should be noted that the data of the thoracic rise and fall here may be a data of a fainted patient obtained by the rescuer, or a data of a patient after rescue have been taken. In the process of cardiopulmonary resuscitation, it is also possible to monitor whether the artificial ventilation is effective or whether the artificial ventilation meets the standard by monitoring the thoracic rise and fall of the patient caused by artificial ventilation. By determining whether the patient is in a status of spontaneous breathing, etc., it is possible to determine the rescue operations that the rescuer needs to take, determine whether further rescue is required, or to evaluate the effectiveness of the rescue.

In other embodiments, the medical device may include a visual indication module. Taking the visual indication module including a laser emitting device as an example, the emitted laser beam will appear as a laser spot when it hits an object. The laser emitting device may be arranged on the central axis of the two lenses 19, in this case, the laser beam is basically consistent with the sight of the rescuer.

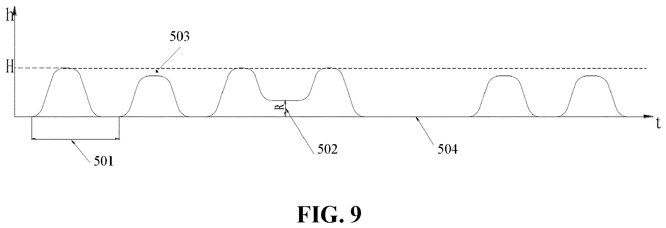

Still taking the electromagnetic wave distance measurement device in FIG. 8 as an example, the laser beam can hit on a back of the rescuer's hand when he is performing CPR chest compression. As the chest compressions progress, the patient's thorax will reciprocate between the depression being pressed and the recoil after being released. The reflection point of the electromagnetic wave 41 emitted by the electromagnetic wave transmitting unit 112a is set to overlap with the laser spot, then positions of the reflection point obtained by the electromagnetic wave distance measurement device are the positions of the back of the rescuer's hand, that is, the passive positions of the patient's thorax during the chest compressions. FIG. 9 is a graph of thoracic rise and fall data of a patient during chest compressions, it shows that when the medical device wearer performs CPR on a patient, after obtaining the data of thoracic rise and fall of the patient during CPR, one or more of data of depth of compressions, rate of compressions, incomplete recoil of compressions, duration of compression interruption and total duration of compressions can be obtained according to the data of thoracic rise and fall during CPR.

Specifically, when a person wearing the medical device performs CPR chest compressions on a patient, in each compression cycle, there is a position with the largest distance and a position with the smallest distance between the patient's thorax and the medical device. The difference between them is the depth of CPR compression, in the waveform diagram of FIG. 9, is the difference between a position corresponding to a wave peak and a position corresponding to the adjacent wave trough. The depth of CPR compression recommended by the AHA guidelines is about 5-6cm. In the actual medical process, the CPR compression depth may fluctuate. As shown in FIG. 9, the patient's chest does not reach the required depth when the rescuer presses, in this case, there is an incomplete recoil of compression R (shown at 503). Of course, in practice, there is also a situation in which the thorax is not fully recoiled, or the compression is not fully released (shown at 502). In addition, a compression interruption (shown at 504) may also occur during the chest compression, that is, the position of the patient's thorax does not change during a certain compression cycle. The waveform at 501 indicates an interval between the starting points of two compressions, that is, the compression cycle, and its reciprocal is the compression rate. The AHA guidelines recommend a compression rate of 100-120 times per minute, or a compression cycle of 0.5-0.6 seconds. In the actual medical process, incomplete recoil of CPR compressions, compression interruptions are relatively common. Through the thoracic rise and fall data of the patient during the CPR operations provided by the medical device of the present disclosure, four parameters of chest compressions required in the clinical medicine can be obtained, and at the same time, it is possible to monitor whether the rescuer's CPR operation is qualified in real time, and accordingly, the diagnosis module or the remote ends of the medical device can make prompts or provide modified rescue program, then the rescuer can improve the rescue operations accordingly, thereby increasing the success rate of the rescue.

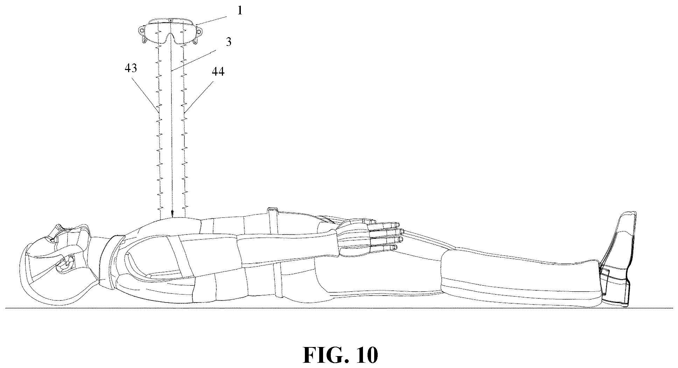

In some other embodiments, the distance measurement module may include a plurality of distance measurement devices. As shown in FIG. 10, the distance measurement module includes a first electromagnetic wave distance measurement device and a second electromagnetic wave distance measurement device. The first electromagnetic wave distance measurement device and the second electromagnetic wave distance measurement device are set so that the electromagnetic waves emitted by them are parallel. A position HI of a thorax of the patient and a position H2 of a thorax of the patient at a moment are acquired respectively through the first electromagnetic wave distance measurement device and the second electromagnetic wave distance measurement device, and a position H of a thorax of the patient at the moment is an average value of the position HI and the position H2.

The distance is measured by two-path electromagnetic waves configured in parallel, and the average value of the two paths is served as the measured distance, which can reduce the measurement error of the distance measurement module and thereby improve the accuracy of the data of thoracic rise and fall of a patient.

During the process of CPR chest compressions, the medical device will generate measurement errors due to the movement of the wearer's head, that is, the movement of the medical device itself will cause changes in the measured value of the distance between the medical device and the reflection point. In practice, the reflection point of one electromagnetic wave distance measurement device of the distance measurement module can be on a fixed object, and the change of its position can represent the change of the displacement of the wearer's head, and another electromagnetic wave distance measurement device is used to measure the position of the patient's chest, the two measurements can be superimposed to obtain the thoracic rise and fall data of the patient. In other embodiments, in order to solve above problem, image recognition technology can be introduced, that is, the information acquisition module of the medical device includes an image capture module, and at the same time, the medical device is configured with a visual indication module that can generate a beam spot. Still taking the visual indication module including a laser emitting device as an example, a laser beam spot is displayed when the emitted laser hits on an object. For a specific laser emitting device, in the physical space, an area SO of the laser beam spot and a distance between the laser emitting device and the laser beam spot have a one-to-one correspondence, that is, there is a certain function between the area SO of the laser beam spot and the distance. In this case, the area SO of the laser beam spot can be calculated from the distance L between the laser emitting device and the beam spot, and vice versa.

In the above embodiment, when the physiological sign information of a patient includes pupil status data, the medical device acquires at least one image of eyeball of the patient;
acquiring at least one image of the beam spot;
obtaining a size of a pupil and a size of an eyeball, and/or pupil-to-eyeball ratio of the patient according to at least one image of the beam spot and the at least one image of eyeball.

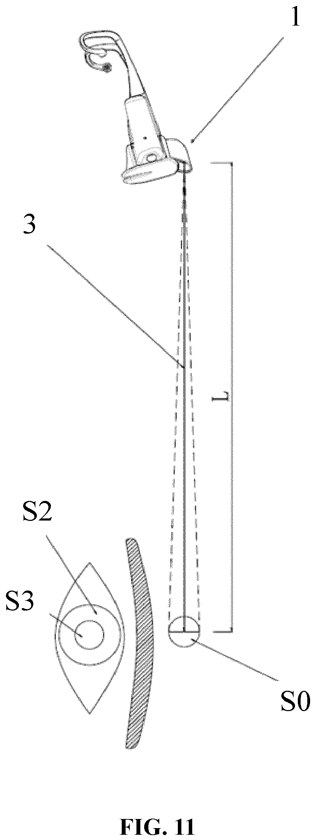

Specifically, as shown in FIG. 11, an image of the laser beam spot can be obtained through the image acquisition module, at the same time, the distance between the laser beam spot and the medical device can be obtained through the distance measurement module of the medical device, and then the area SO of the laser beam spot in the actual physical space can be obtained according the distance. Thus, the corresponding relationship between the area of the laser beam spot in the image system and the area SO of the laser beam spot in the physical space is established through image recognition technology. Then, an area of the pupil and an area of the eyeball of the patient in the image system are obtained from the eyeball images respectively through image recognition technology, and pupil size S2 and eyeball size S3 of the patient are obtained through the established correspondence relationship between the image system and the physical space. Medically, the normal pupil size is 2-3cm, and the dilated pupil size is 3-5cm. When the diagnostic module of the medical device determines that the obtained pupil size of the patient is larger than the medical normal value, it can be considered that the patient is in a status of pupil dilation. In medical practice, whether the pupil/eyeball ratio is greater than a second threshold can also be used to determine whether the patient is in a status of pupil dilation. Similarly, the second threshold here may be empirical data obtained clinically. The pupil/eyeball ratio can be calculated from the pupil size and eyeball size obtained by the above method, and the pupil/eyeball ratio can be determined by obtaining the area of the pupil and the area of the eyeball in the image system from the eyeball image through image recognition technology.

In this embodiment, when the physiological sign information includes the patient's thoracic rise and fall data, the step of acquiring the patient's physiological sign information by the medical device includes:
acquiring a plurality of thoracic images of a patient in a period;
obtaining a data of thoracic rise and fall during the period according to the plurality of thoracic images.

Specifically, the distance between the thorax and the medical device can be estimated through the change in an area of the specific indicator of the thoracic image. Ideally, the coordinates of the physical space where a patient is located can be constructed through the image acquisition system and the visual indication module of the medical device, and the measurement errors due to the movement of the medical device or other reasons can be eliminated through the distance measurement of fixed objects in the environment. For example, through the image acquisition module and image recognition technology, continuous images of a fixed object are obtained, and the position change ΔH of the head mounted medical device is calculated through the change of the pixel block of the fixed object (static feature). The position of patient's thorax obtained by the distance measurement module can be corrected with the position change ΔH, which will reduce the measurement errors caused by the movement of the wearer's head, further improve the accuracy of physiological sign information such as the data of thoracic rise and fall and/or pupil status etc., and ultimately improve the accuracy of diagnosis.

In the embodiment in which the medical device is configured with an image capture module, the medical device may further include a diagnosis module, where the diagnosis module stores a skin status database including a mapping relationship between a skin image and a skin status; the physiological sign information includes skin status data; the step of acquiring the physiological sign information by the medical device includes:
obtaining at least one skin image of a patient;
obtaining skin status data of the patient according to the at least one skin image information through the diagnosis module.
determining whether the patient has cyanosis according to skin status data of the patient.

Of course, the information acquisition module may further include a positioning module, such as a medical device equipped with a GPS navigation or a BeiDou navigation, which is configured to capture geographic location information of the person wearing the medical device. The method may further include: sending the geographic location information to the external wireless communication module. Through the above step, the remote ends can dispatch substances according to the obtained location information. Typically, such as sending an AED by a drone to the rescue site, during this process, the drone can use the location information of the medical device as the destination to accurately navigate to the rescue site. Similarly, other substances such as ambulances, drones that deliver medicines, etc. can also share and obtain the geographic location information of the rescue site.

The above descriptions of the present disclosure are given in connection with some specific and preferred embodiments, other embodiments within the scope of the concept of the present disclosure are not limited to the above descriptions. Modifications and substitutions can be made without departing from the spirit and scope of the present disclosure.

## Claims

1. A head mounted medical device comprising:
an information acquisition module (M10) for acquiring physiological sign information of a patient and/or operation information of a person wearing the medical device;
a remote communication module (M20) for communicating with an external wireless communication module, wherein, the communicating comprises transmitting a data collected by the information acquisition module (M10) to the external wireless communication module and receiving a data sent by the external wireless transmission module;
an information output module (M30) for displaying or sending the data sent by the external wireless communication module to the person wearing the medical device.

2. The head mounted medical device according to claim 1, wherein the physiological sign information comprises one or more of data of thoracic rise and fall, pupil status and skin status;
the operation information comprises one or more of data of depth of compressions, rate of compressions, incomplete recoil of compressions, duration of compression interruption and total duration of compressions.

3. The head mounted medical device according to claim 1, wherein the information acquisition module (M10) comprises one or more of a distance measurement module (M110), an image capture module (M120) , an audio capture module (M130) and a positioning module (M140), and the positioning module (M140) is configured to capture geographic location information of the person wearing the medical device.

4. The head mounted medical device according to claim 1, wherein the medical device further comprises:
a diagnosis module (M40), configured to receive the data collected by the information acquisition module (M10) and then make a diagnosis and/or give a treatment plan according to the data;
the diagnosis module (M40), comprises a first algorithm model and/or a trained second algorithm model;
the information acquisition module (M10) further comprises an image capture module (M120);
the first algorithm model is configured to determine whether a patient has cyanosis according to at least one skin image of the patient collected by the image capture module (M120);
the second algorithm model is configured to determine whether a patient is in a status of pupil dilation according to at least one eyeball image of the patient collected by the image capture module (M120).

5. The head mounted medical device according to claim 1, wherein the information output module (M30) comprises an image output module (M310) and/or an audio output module (M320); and/or
a warning module (M330), which is configured to send warning information by touching a body of the person wearing the medical device.

6. The head mounted medical device according to claim 1, wherein the medical device further comprises a lighting module (M50) for providing a light source; and/or
a visual indication module (M60) for indicating the location of the data collected by the information acquisition module (M10).

7. The head mounted medical device according to claim 1, wherein the medical device (1) is a device in a form of glasses;
the medical device (1) in the form of glasses comprises lenses (19) and temples (18); the information acquisition module (M10) comprises a distance measurement module (M110), and the distance measurement module (M110) comprises at least one electromagnetic wave distance measurement device, and the at least one electromagnetic wave distance measurement device is arranged around the lenses (19); and/or
the information acquisition module (M10) comprises an image capture module (M120), and the image capture module (M120) comprises two cameras, and the cameras are respectively arranged around the lenses (19); and/or
the information acquisition module (M10) comprises an audio capture module (M130), and the audio capture module (M130) comprises at least one sound pickup (113), and the at least one sound pickup (113) is arranged on the temples (18) or around the lenses (19).

8. The head mounted medical device according to claim 7, wherein the information output module (M30) comprises an image output module (M310), the image output module (M310) comprises a display (131) , the display (131) is moved to a position of the lens (19) when the medical device outputs an image; and/or
the information output module (M30) comprises an audio output module (M320), and the audio output module (M320) comprises at least one speaker (132), and the speaker (132) is arranged on the temples (18); and/or
the information output module (M30) comprises a warning module (M330), and the warning module (M330) comprises at least one vibrator, and the at least one vibrator is arranged on the temples (18).

9. A method for operating a head mounted medical device comprising steps of:
the medical device acquiring the physiological sign information of a patient and/or the operation information of a person wearing the medical device;
the medical device sending the physiological sign information and/or the medical operation information to an external wireless communication module;
the medical device receiving the data sent by the external wireless communication module, and displaying or sending the data to the person wearing the medical device.

10. The method according to claim 9, wherein the information acquisition module comprises a distance measurement module, and the distance measurement module comprises at least one electromagnetic wave distance measurement device;
the physiological sign information comprises a data of thoracic rise and fall, and the step of acquiring the physiological sign information of a patient by the medical device comprises:
acquiring positions H of a thorax of the patient at a plurality of moments in a period by using the at least one electromagnetic distance measurement device;
obtaining a data of thoracic rise and fall during the period according to positions H of a thorax of the patient; and/or
the distance measurement module comprises a first electromagnetic wave distance measurement device and a second electromagnetic wave distance measurement device;
a position HI of a thorax of the patient and a position H2 of a thorax of the patient at a moment respectively are acquired through the first electromagnetic wave distance measurement device and the second electromagnetic wave distance measurement device;
a position H of a thorax of the patient at the moment is an average value of the position HI and the position H2.

11. The method according to claim 10, wherein after obtaining a data of thoracic rise and fall during the period, the method further comprises the following steps:
acquiring two positions of the thorax of the patient at two adjacent moments from the data of thoracic rise and fall;
determining whether a difference between the two positions is greater than a first threshold;
if it is greater than the first threshold, the patient is considered to be in a status of spontaneous breathing;
if it is not greater than the first threshold, the patient is considered to be in a status of cardiac arrest.

12. The method according to claim 10, wherein when a person wearing the medical device performs CPR on a patient, the method further comprises:
acquiring a data of thoracic rise and fall during CPR;
obtaining one or more of data of depth of compressions, rate of compressions, incomplete recoil of compressions, duration of compression interruption and total duration of compressions according to the data of thoracic rise and fall during CPR.

13. The method according to claim 9, wherein the information acquisition module comprises an image capture module, and the image capture module comprises at least one camera;
the physiological sign information comprises a data of thoracic rise and fall, and the step of acquiring the physiological sign information of a patient by the medical device comprises:
acquiring a plurality of thoracic images of a patient in a period;
obtaining a data of thoracic rise and fall during the period according to the plurality of thoracic images; and/or
the information acquisition module comprises an image capture module, and the image capture module comprises at least one camera;
the physiological sign information comprises a data of pupil status, and the step of acquiring the physiological sign information of a patient by the medical device comprises:
acquiring at least one image of eyeball of a patient in a period;
obtaining a pupil-to-eyeball ratio of the patient according to the at least one image of eyeball.

14. The method according to claim 13, wherein the medical device further comprises a visual indication module, and the visual indication module emits a beam spot, the method further comprises:
acquiring at least one image of the beam spot and at least one image of eyeball of a patient;
obtaining a size of a pupil and a size of an eyeball, and/or pupil-to-eyeball ratio of the patient according to at least one image of the beam spot and the at least one image of eyeball; and/or
the information acquisition module further comprises a positioning module, which is configured to capture geographic location information of the person wearing the medical device;
the method further comprises:
sending the geographic location information to the external wireless communication module.

15. The method according to claim 9, wherein the information acquisition module comprises an image capture module, and the image capture module comprises at least one camera;
the medical device further comprises a diagnosis module, and the diagnosis module stores a skin status database including a mapping relationship between a skin image and a skin status;
the physiological sign information comprises skin status data; the step of acquiring the physiological sign information by the medical device comprises:
obtaining at least one skin image of a patient;
obtaining skin status data of the patient according to the at least one skin image information through the diagnosis module.
